# EUROPEAN PATENT APPLICATION

(11) **EP 4 817 640 A1**
(43) Date of publication of application: **07.10.2026**
(21) Application number: 24791960.8
(22) Date of filing: 15.04.2024
(51) Int. Cl.: A61L 15/24, A61F 13/15, A61F 13/534, A61L 15/42, A61L 15/48, A61L 15/20

(54) **METHOD FOR PREPARING ABSORBENT CORE OF LIQUID SANITARY NAPKIN**

(30) Priority: 17.04.2023 CN 202310408413
(71) Applicant: The Procter & Gamble Company, Cincinnati, OH 45202 (US)
(72) Inventor: FAN, Jianquan, Guangzhou, Guangdong 510800 (CN); XIONG, Yuzi, Guangzhou, Guangdong 510800 (CN)
(74) Representative: P&G Patent Germany
(86) International application number: PCT/CN2024/087738
(87) International publication number: WO 2024/217375

(57) **Abstract**

Provided in the present invention is a method for preparing an absorbent core of a liquid sanitary napkin. The preparation method comprises the following steps: S1, mixing an acrylate monomer 1, a cross-linking agent, a photoinitiator and a surfactant to obtain an oil phase 1, and then adding a water phase A and a water phase B thereto to obtain an HIPE emulsion 1; S2, mixing an acrylate monomer 2, a cross-linking agent, a photoinitiator and a surfactant to obtain an oil phase 2, and then adding a water phase C and a water phase D thereto to obtain an HIPE emulsion 2; and S3, coating a piece of cloth with the HIPE emulsion 1 and the HIPE emulsion 2, subjecting same to illumination and steam treatment, and then dewatering, drying and slitting same to obtain an absorbent core of a liquid sanitary napkin, wherein the surfactant is obtained by reacting an aminoalkyl acrylate with an alkyl halide and/or an alkyl halide derivative. The present invention improves the transfer capacity of liquid, enables the product to have the effects of efficient absorption and long-lasting dryness, overcomes defects in the prior art, and has good application prospects.

## Description

### TECHNICAL FIELD

The present invention relates to the technical field of foam-based absorbent cores, and particularly relates to a method for preparing an absorbent core of a liquid sanitary napkin.

### BACKGROUND

The so-called "liquid sanitary napkin" derives its name because the raw material of its absorbent core is derived from a liquid material, and the product has the characteristics of lightness and thinness, breathability, imperceptible tactile sensation, dryness, fast absorption and the like compared with traditional sanitary napkins. Although it is called the liquid sanitary napkin, it actually does not contain liquid or cotton. Instead, it is a microporous absorbent material made by employing a new technology, and this absorbent core is characterized in that the material is full of pores (high porosity), light and thin and easy to breathe, and the material can spontaneously absorb fluid, thereby avoiding the risk of precipitation of SAP particles in the traditional sanitary napkin products.

However, most of the existing liquid sanitary napkin products have many problems such as side leakage and being prone to rewet, and have significant defects in their absorption effect. These products cannot efficiently transfer the absorbed liquid, making it difficult to achieve the effects of fast absorption and long-lasting dryness. Thus, they can no longer meet the use needs of the market and users.

In summary, there is an urgent need to develop a new technical solution to solve the problems in the prior art and meet the development needs of the current market.

### SUMMARY

An "HIPE emulsion" in the present invention refers to a high internal phase emulsion.

Based on this, an acrylic ester monomer is mixed with a cross-linking agent, an initiator and a specific surfactant to obtain an absorbent core with stronger absorption performance after reaction, and also, the upper and lower layers of the absorbent core are configured with different pore sizes to enable a good capillary action of the product. Consequently, the present invention further improves the liquid transfer capacity, enables the product to have the effects of efficient absorption and long-lasting dryness, overcomes the defects in the prior art, and has good application prospects.

An object of the present invention is to provide a method for preparing an absorbent core of a liquid sanitary napkin, comprising the steps of:
S1. mixing an acrylic ester monomer 1, a cross-linking agent, a photoinitiator and a surfactant to obtain an oil phase 1, and then adding a water phase A comprising an inorganic salt and a water phase B comprising an initiator and acrylate and/or propylene sulfonate and a derivative thereof to obtain an HIPE emulsion 1;
S2. mixing an acrylic ester monomer 2, a cross-linking agent, a photoinitiator and a surfactant to obtain an oil phase 2, and then adding a water phase C comprising an inorganic salt and a water phase D comprising an initiator and acrylate and/or propylene sulfonate and a derivative thereof to obtain an HIPE emulsion 2;
S3. coating the HIPE emulsion 1 and the HIPE emulsion 2 on the upper and lower surfaces of a fabric respectively, subjecting same to illumination and steam treatment, and then dehydrating and drying same to obtain the absorbent core of the liquid sanitary napkin;
wherein,
the surfactant is obtained by reacting acrylic amino alkyl ester and/or an acrylic amino alkyl ester derivative with a haloalkane and/or a haloalkane derivative;
the inorganic salt is selected from one or more of alkaline earth metal chloride or sulfate and alkali metal chloride or sulfate;
the fabric is selected from one of woven fabric and non-woven fabric.

Specifically, the acrylic amino alkyl ester of the present invention generally refers to acrylic amino alkyl ester having two alkyl segments attached thereto, including but not limited to dimethylaminoalkyl (meth) acrylate, diethylaminoalkyl (meth) acrylate, dipropylaminoalkyl (meth) acrylate, dibutylaminoalkyl (meth) acrylate, and the like.

Further, in the acrylic amino alkyl ester and/or the acrylic amino alkyl ester derivative, the alkyl group in the alkyl ester is selected from a C1-C20 alkyl group; the amino group is -N(R₁)(R₂), wherein the R₁ and R₂ are independently selected from a hydrogen atom or a C1-C20 alkyl group;
in the acrylic amino alkyl ester derivative,

One or more of a fluorine atom, a chlorine atom, a bromine atom, an iodine atom or a C1-C20 alkyl group are substituted for one or more hydrogen atoms on the acrylic group;
and/or
one or more of polyethylene glycol, polypropylene glycol, and polybutylene glycol segments are substituted for one or more carbon atoms in the alkyl group;
   and/or
a C = C double bond on the acrylic group can be reduced, oxidized or added to form different groups.

Further, the preparation method of the surfactant comprises the steps of:
adding acrylic amino alkyl ester and/or an acrylic amino alkyl ester derivative, a haloalkane and/or a haloalkane derivative, and a polymerization inhibitor to a solvent, reacting them with heating and stirring at 40-60°C for 20-30h, and then subjecting same to purification to obtain the surfactant.

The surfactant prepared by the present invention can form micelles in solution, significantly reducing the surface tension and exhibiting good emulsifying ability, thereby promoting a more uniform and stable dispersion of each component in the emulsion in the system. It is surprisingly found that the surfactant does not affect the cross-linking structure as an impurity in the polymerization reaction, and has a better effect than conventional surfactants. Since the component contains an acrylic structure, it can participate in the polymerization reaction. The technical effect of regulating the pore size of the absorbent core can be achieved by adjusting the content of each component and reacting with the acrylate, the acrylic monomer having a long-chain structure and the cross-linking agent. By providing the upper and lower layers of the absorbent core with large pores and small pores respectively, capillary action is achieved, which enables fast penetration of liquid to the lower layer and fast and even dispersion and storage of blood in the bottom layer, and prevents blood accumulation on the surface layer of the sanitary napkin, thereby effectively solving the problem of side leakage.

Further, in the haloalkane and/or the haloalkane derivative, the halo atom is selected from one of a fluorine atom, a chlorine atom, a bromine atom, and an iodine atom; the number of the carbon atoms of the alkyl group is selected from 14-25;
in the haloalkane derivative, one or more of a hydrogen atom, an oxygen atom, alkenyl, alkynyl, aryl, hydroxyl, amino, carbonyl, carboxyl, an ester group, cyano, and a nitro group are substituted for one or more carbon atoms on the alkyl group;
   and/or
in the haloalkane derivative, one or more of a fluorine atom, a chlorine atom, a bromine atom and an iodine atom are substituted for one or more hydrogen atoms on the alkyl group;
   and/or
in the haloalkane derivative, one or more of a sulfonate group and a methyl sulfate group are substituted for the halogen atom.

Further,
in step S1, a mass ratio of the acrylic ester monomer 1, the cross-linking agent, the photoinitiator, and the surfactant is (40-100):(5-40):(0.05-2):(2-10); in the water phase A, the mass fraction of the inorganic salt is 1-4%; in the water phase B, the mass fraction of the initiator is 0.1-10%, and the mass fraction of the acrylate and/or the propylene sulfonate and the derivative thereof is 0.1-5%; a volume ratio of the oil phase 1, the water phase A and the water phase B is 1:(20-40):(1-10);
in step S2, a mass ratio of the acrylic ester monomer 2, the cross-linking agent, the photoinitiator, and the surfactant is (40-100):(5-40):(0.05-2):(2-10); in the water phase C, the mass fraction of the inorganic salt is 1-4%; in the water phase D, the mass fraction of the initiator is 0.1-10%, and the mass fraction of the acrylate and/or the propylene sulfonate and the derivative thereof is 0.1-5%; a volume ratio of the oil phase 2, the water phase C and the water phase D is 1:(28-40):(1-10).

Further, the HIPE emulsion 1 and the HIPE emulsion 2 are made using a continuous emulsifying apparatus.

Further, the acrylic ester monomer 1 and the acrylic ester monomer 2 are alkyl acrylate or alkyl methacrylate, and the alkyl group in the alkyl acrylate or alkyl methacrylate is selected from a C4-C20 alkyl group.

Further, the cross-linking agent is selected from one or more of ethylene glycol diacrylate, ethylene glycol dimethacrylate, butylene glycol dimethacrylate, butylene glycol diacrylate, hexylene glycol diacrylate, hexylene glycol dimethacrylate, trimethylol triacrylate, tripropylene glycol diacrylate, trimethylol trimethacrylate, and tripropylene glycol dimethacrylate.

Further, in step S3, the illumination has a wavelength of 205-450 nm, an intensity of 40-1000 mW/cm², and a duration of 5-180 s.

Further, the initiator is selected from one or more of ammonium persulfate, sodium persulfate, potassium persulfate, and an azo initiator.

Further, the photoinitiator is preferably one or more of benzophenone, benzil, thioxanthone, benzyl ketal, α - hydroxyalkylphenone, α - aminoalkylphenone and acylphosphine oxide.

Further, the acrylate and/or propylene sulfonate includes sodium acrylate, sodium methacrylate, sodium methyl propylene sulfonate and sodium propylene sulfonate, and the parts by mass of the sodium acrylate, sodium methacrylate, sodium methyl propylene sulfonate and sodium propylene sulfonate are 0.1-50 parts.

Specifically, the present invention relates to the following embodiments:
1. A method for preparing an absorbent core of a liquid sanitary napkin, comprising the steps of:
   a. mixing a monomer, a cross-linking agent, a photoinitiator, and a surfactant to form an oil phase;
   b. adding a water phase to the oil phase to form a first high internal phase emulsion;
   c. depositing the first high internal phase emulsion on a surface;
   d. exposing the first high internal phase emulsion to a light treatment;
   e. placing the first high internal phase emulsion in a steam tunnel furnace;
   f. dehydrating and drying the high internal phase emulsion to obtain the absorbent core;
   wherein the surfactant is obtained by reacting acrylic amino alkyl ester and/or an acrylic amino alkyl ester derivative with a haloalkane and/or a haloalkane derivative.
2. The method of embodiment 1, further comprising mixing a second monomer, a second cross-linking agent, a second photoinitiator, and a second surfactant to form a second oil phase; and adding a second water phase to the second oil phase to form a second high internal phase emulsion.
3. The method of embodiment 2, wherein the second high internal phase emulsion is deposited on the first high internal phase emulsion.
4. The method of any one of the preceding embodiments, wherein the alkyl group in the alkyl ester is selected from C1-C20.
5. The method of any one of the preceding embodiments, wherein the amino group is - N(R₁)(R₂); wherein R₁ and R₂ are independently selected from a hydrogen atom or a C1-C20 alkyl group.
6. The method of any one of the preceding embodiments, wherein the monomer is an acrylic ester monomer.
7. The method of any one of the preceding embodiments, wherein the monomer is selected from alkyl acrylate or alkyl methacrylate.
8. The method of embodiment 2, wherein the second monomer is selected from isooctyl acrylate or isooctyl methacrylate.
9. The method of any one of the preceding embodiments, wherein the light treatment comprises exposing the first high internal phase emulsion to a light having a wavelength of from 205 nm to 450 nm.
10. The method of any one of the preceding embodiments, wherein the water phase comprises an inorganic salt selected from one or more of alkaline earth metal chloride or sulfate and alkali metal chloride or sulfate.
11. The method of any one of the preceding embodiments, wherein the first high internal phase emulsion is made using a continuous emulsifying apparatus.
12. The method of any one of the preceding embodiments, wherein the cross-linking agent is selected from the group consisting of: ethylene glycol diacrylate, ethylene glycol dimethacrylate, butylene glycol diacrylate, hexylene glycol dimethacrylate, trimethylol triacrylate, tripropylene glycol diacrylate, trimethylol trimethacrylate, tripropylene glycol dimethacrylate, and combinations thereof.
13. The method of any one of the preceding embodiments, wherein the oil phase comprises a mass ratio of the monomer to the cross-linking agent to the photoinitiator to the surfactant of 40-100: 5-40: 0.05-2: 2-10.
14. The method of any one of the preceding embodiments, wherein the water phase comprises from 0.1 to 10 wt% of an initiator.
15. The method of embodiment 14, wherein the initiator is selected from one or more of ammonium persulfate, sodium persulfate, potassium persulfate, and an azo initiator.
16. The method of any one of the preceding embodiments, wherein the photoinitiator is one or more of benzophenone, benzil, thioxanthone, benzyl ketal, α - hydroxyalkyl benzophenone, α - aminoalkylbenzophenone, and acylphosphine oxide.
17. A high internal phase emulsion, comprising an oil phase comprising a monomer, a cross-linking agent, a photoinitiator, and a surfactant; and a water phase; wherein the surfactant is obtained by reacting acrylic amino alkyl ester and/or an acrylic amino alkyl ester derivative with a haloalkane and/or a haloalkane derivative.
18. The high internal phase emulsion of embodiment 17, wherein the alkyl group in the alkyl ester is selected from C1-C20.
19. The high internal phase emulsion of embodiment 17 or 18, wherein the amino group is -N(R₁)(R₂); wherein R₁ and R₂ are independently selected from a hydrogen atom or a C1-C20 alkyl group.
20. The high internal phase emulsion of any one of embodiments 17 to 19, wherein the monomer is an acrylic ester monomer.
21. The high internal phase emulsion of any one of embodiments 17 to 19, wherein the monomer is selected from alkyl acrylate or alkyl methacrylate.
22. The high internal phase emulsion of any one of embodiments 17 to 21, wherein the water phase comprises an inorganic salt selected from one or more of alkaline earth metal chloride or sulfate and alkali metal chloride or sulfate.
23. The high internal phase emulsion of any one of embodiments 17 to 22, wherein the cross-linking agent is selected from the group consisting of: ethylene glycol diacrylate, ethylene glycol dimethacrylate, butylene glycol diacrylate, hexylene glycol dimethacrylate, trimethylol triacrylate, tripropylene glycol diacrylate, trimethylol trimethacrylate, tripropylene glycol dimethacrylate, and combinations thereof.
24. The high internal phase emulsion of any one of embodiments 17 to 23, wherein the oil phase comprises a mass ratio of the monomer to the cross-linking agent to the photoinitiator to the surfactant of 40-100: 5-40: 0.05-2: 2-10.
25. The high internal phase emulsion of any one of embodiments 17 to 24, wherein the water phase comprises from 0.1 to 10 wt% of an initiator.

The beneficial effects of the present invention are as follows:
1. The present invention uses a specific surfactant to be compounded with a long-chain acrylic ester monomer and a cross-linking agent to prepare an absorbent core with stronger adsorption performance according to the HIPE synthesis process and method, so that the product has a higher porosity and a dense microporous structure. Consequently, the present invention achieves the 3D stereoscopic fluid storage functionality, maintains dryness on the surface of the sanitary napkin, prolongs the wear time of the sanitary napkin, and solves the problems of blood rewet and side leakage under pressure of the traditional core materials.
2. The present invention optimizes the design about the pore sizes of the material layers of the core according to the fluid characteristics of human blood, such that the upper layer of the absorbent core is an absorbent layer with a large pore size of 30-60 µm, which is suitable for the fast absorption of fluid and allows fast penetration of the liquid to the lower layer; and the lower layer is an absorbent foam layer with a small pore size of 1-15 µm, which is suitable for the dispersion and storage of the water-containing fluid and allows fast and even dispersion of the fluid in the lower layer. Moreover, according to the capillary action principle, since the size of the pores of the lower layer is significantly smaller than that of the pores of the upper layer, the small pores of the lower layer of the core can more efficiently transfer and absorb the fluid from the upper layer, which keeps the upper layer dry.

### BRIEF DESCRIPTION OF THE DRAWINGS

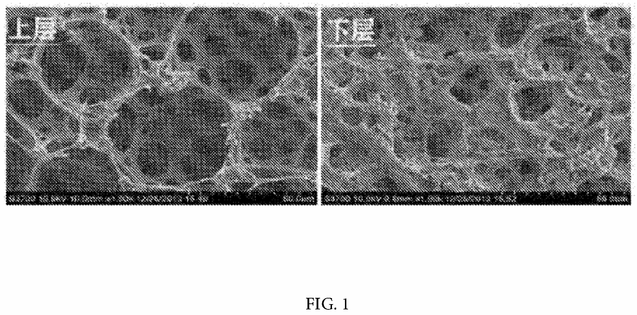

FIG. 1 is an SEM image of an upper layer and a lower layer in an absorbent core of a liquid sanitary napkin prepared in Example 1 of the present invention.

### DETAILED DESCRIPTION OF EMBODIMENTS

In order to more clearly illustrate the technical solutions of the present invention, the following examples are listed. The raw materials, reactions and post-treatment means appearing in the examples, are all common raw materials on the market and technical means well known to those skilled in the art, unless otherwise specified.

An initiator in the examples of the present invention is ammonium persulfate.

A photoinitiator in the examples of the present invention is 1-hydroxycyclohexyl phenyl ketone (photoinitiator 184).

The "parts" in the examples of the present invention all refer to parts by mass.

### Example 1

A method for preparing an absorbent core of a liquid sanitary napkin, comprising the steps of:
S0. adding dimethylaminoethyl methacrylate, 1-chlorodocosane (dimethylaminoethyl methacrylate:1-chlorodocosane = 1:1, n/n) and a trace amount of a polymerization inhibitor (phenothiazine) to acetone, reacting them with stirring at 50°C for 20 h, and then subjecting same to centrifugation, washing and drying to obtain a surfactant;
S1. mixing 55 parts of eicosyl methacrylate, 20 parts of ethylene glycol dimethacrylate, 1 part of a photoinitiator and 5 parts of the surfactant to obtain an oil phase 1, and then adding a water phase A containing 2% by mass of calcium chloride and a water phase B containing 7% by mass of an initiator, 0.5% by mass of sodium acrylate, 0.5% by mass of sodium methacrylate, 0.5% by mass of sodium methyl propylene sulfonate, and 0.5% by mass of sodium propylene sulfonate, with a volume ratio of the oil phase 1, the water phase A and the water phase B being 1:26:1, to obtain an HIPE emulsion 1;
specifically, in a continuous emulsifying apparatus, the oil phase was prepared in an oil phase material storage tank, the inorganic salt water phase was prepared in a water phase material storage tank, and the initiator water phase was prepared in an initiator storage tank, and during stable operation of the emulsifying apparatus, part of the emulsion from a primary mixing and emulsification in a first static mixer module was delivered back to a first emulsification kettle by the control of a first back flow constant-flow pump, and part of the emulsion from a secondary mixing and emulsification in a second static mixer module was delivered back to a second emulsification kettle by the control of a second back flow constant-flow pump; the flow rate of the back flow is precisely controlled by the first back flow constant-flow pump, and at the same time, the oil phase in the oil phase material storage tank and the water phase in the water phase material storage tank were continuously delivered to the first emulsification kettle by an oil phase feed constant-flow pump and a water phase feed constant-flow pump respectively, and were premixed with the emulsion being mixed and emulsified in the first emulsification kettle to obtain a premixed material, which was delivered to the first static mixer module for mixing and emulsification to obtain a premixed emulsion 1 with a water-oil ratio of 14:1;
the premixed emulsion 1 was partially delivered to the first emulsification kettle under the control of the first back flow constant-flow pump and partially delivered to the second emulsification kettle under the control of a premixed emulsion 1 feed constant-flow pump, and the premixed emulsion 1 in the premixed emulsion 1 feed constant-flow pump and the water phase continuously delivered to the second emulsification kettle by the water phase feed constant-flow pump were premixed to obtain a premixed material, which was delivered to the second static mixer module for mixing and emulsification to obtain a premixed emulsion 2; the premixed emulsion 2 was partially delivered to the second emulsification kettle under the control of the second back flow constant-flow pump and partially delivered to a third static mixer module under the control of a constant-flow pump, and was finally mixed and emulsified with the initiator water phase continuously delivered under the control of an initiator feed constant-flow pump, to obtain an HIPE emulsion 1, which was continuously delivered to the next process via an emulsified product output interface;
S2. mixing 40 parts of isooctyl acrylate, 10 parts of ethylene glycol dimethacrylate, 1 part of a photoinitiator and 2 parts of the surfactant to obtain an oil phase 2, and then adding a water phase C containing 1% by mass of calcium chloride and a water phase D containing 8% by mass of the initiator, 0.5% by mass of sodium acrylate, 0.5% by mass of sodium methacrylate, 0.5% by mass of sodium methyl propylene sulfonate, and 0.5% by mass of sodium propylene sulfonate, with a volume ratio of the oil phase 2, the water phase C and the water phase D being 1:31:1, to obtain an HIPE emulsion 2;
the treatment process of the HIPE emulsion 2 in the continuous emulsifying apparatus was the same as that in S1;
S3. coating the HIPE emulsion 1 on the lower surface of the non-woven fabric with a thickness of 1.5 mm; coating the HIPE emulsion 2 on the upper surface of the non-woven fabric with a thickness of 0.5mm; and then using the illumination with a wavelength of 450 nm and an intensity of 50 mW/cm² for 120 s, followed by curing in a steam tunnel furnace for 5 min, and then dehydrating, drying and slitting to obtain the absorbent core of the liquid sanitary napkin.

FIG. 1 is an SEM image of the upper layer and the lower layer in the absorbent core of the liquid sanitary napkin prepared in Example 1.

### Example 2

A method for preparing an absorbent core of a liquid sanitary napkin, comprising the steps of:
S0. adding dimethylaminoethyl methacrylate, 1-chloroeicosane (dimethylaminoethyl methacrylate:1-chloroeicosane = 1:1, n/n) and a trace amount of a polymerization inhibitor (phenothiazine) to acetone, reacting them with stirring at 45°C for 20 h, and then subjecting same to centrifugation, washing and drying to obtain a surfactant;
S1. mixing 60 parts of octadecyl acrylate, 25 parts of ethylene glycol dimethacrylate, 1 part of a photoinitiator and 8 parts of the surfactant to obtain an oil phase 1, and then adding a water phase A containing 2% by mass of calcium chloride and a water phase B containing 7% by mass of an initiator, 0.5% by mass of sodium acrylate, 0.5% by mass of sodium methacrylate, 0.5% by mass of sodium methyl propylene sulfonate, and 0.5% by mass of sodium propylene sulfonate, with a volume ratio of the oil phase 1, the water phase A and the water phase B being 1:30:1, to obtain an HIPE emulsion 1;
specifically, in a continuous emulsifying apparatus, the oil phase was prepared in an oil phase material storage tank, the inorganic salt water phase was prepared in a water phase material storage tank, and the initiator water phase was prepared in an initiator storage tank, and during stable operation of the emulsifying apparatus, part of the emulsion from a primary mixing and emulsification in a first static mixer module was delivered back to a first emulsification kettle by the control of a first back flow constant-flow pump, and part of the emulsion from a secondary mixing and emulsification in a second static mixer module was delivered back to a second emulsification kettle by the control of a second back flow constant-flow pump; the flow rate of the back flow is precisely controlled by the first back flow constant-flow pump, and at the same time, the oil phase in the oil phase material storage tank and the water phase in the water phase material storage tank were continuously delivered to the first emulsification kettle by an oil phase feed constant-flow pump and a water phase feed constant-flow pump respectively, and were premixed with the emulsion being mixed and emulsified in the first emulsification kettle to obtain a premixed material, which was delivered to the first static mixer module for mixing and emulsification to obtain a premixed emulsion 1 with a water-oil ratio of 14:1;
the premixed emulsion 1 was partially delivered to the first emulsification kettle under the control of the first back flow constant-flow pump and partially delivered to the second emulsification kettle under the control of a premixed emulsion 1 feed constant-flow pump, and the premixed emulsion 1 in the premixed emulsion 1 feed constant-flow pump and the water phase continuously delivered to the second emulsification kettle by the water phase feed constant-flow pump were premixed to obtain a premixed material, which was delivered to the second static mixer module for mixing and emulsification to obtain a premixed emulsion 2; the premixed emulsion 2 was partially delivered to the second emulsification kettle under the control of the second back flow constant-flow pump and partially delivered to a third static mixer module under the control of a constant-flow pump, and was finally mixed and emulsified with the initiator water phase continuously delivered under the control of an initiator feed constant-flow pump, to obtain an HIPE emulsion 1, which was continuously delivered to the next process via an emulsified product output interface;
S2. mixing 50 parts of isooctyl methacrylate, 20 parts of ethylene glycol dimethacrylate, 1 part of a photoinitiator and 5 parts of the surfactant to obtain an oil phase 2, and then adding a water phase C containing 1% by mass of calcium chloride and a water phase D containing 6% by mass of the initiator, 0.5% by mass of sodium acrylate, 0.5% by mass of sodium methacrylate, 0.5% by mass of sodium methyl propylene sulfonate, and 0.5% by mass of sodium propylene sulfonate, with a volume ratio of the oil phase 2, the water phase C and the water phase D being 1:28:1, to obtain an HIPE emulsion 2;
the treatment process of the HIPE emulsion 2 in the continuous emulsifying apparatus was the same as that in S1;
S3. coating the HIPE emulsion 1 on the lower surface of the non-woven fabric with a thickness of 1.5 mm; coating the HIPE emulsion 2 on the upper surface of the non-woven fabric with a thickness of 0.5mm; and then using the illumination with a wavelength of 350 nm and an intensity of 40 mW/cm² for 10 s, followed by curing in a steam tunnel furnace for 5 min, and then dehydrating, drying and slitting to obtain the absorbent core of the liquid sanitary napkin.

### Example 3

A method for preparing an absorbent core of a liquid sanitary napkin, comprising the steps of:
S0. adding dimethylaminoethyl methacrylate, 1-chlorodocosane (dimethylaminoethyl methacrylate:1-chlorodocosane = 1:1, n/n) and a trace amount of a polymerization inhibitor (phenothiazine) to acetone, reacting them with stirring at 40°C for 25 h, and then subjecting same to centrifugation, washing and drying to obtain a surfactant;
S1. mixing 80 parts of eicosyl methacrylate, 40 parts of ethylene glycol dimethacrylate, 1 part of a photoinitiator and 8 parts of the surfactant to obtain an oil phase 1, and then adding a water phase A containing 3% by mass of calcium chloride and a water phase B containing 10% by mass of an initiator, 0.4% by mass of sodium acrylate, 0.4% by mass of sodium methacrylate, 0.1% by mass of sodium methyl propylene sulfonate, and 0.1% by mass of sodium propylene sulfonate, with a volume ratio of the oil phase 1, the water phase A and the water phase B being 1:20:1, to obtain an HIPE emulsion 1;
specifically, in a continuous emulsifying apparatus, the oil phase was prepared in an oil phase material storage tank, the inorganic salt water phase was prepared in a water phase material storage tank, and the initiator water phase was prepared in an initiator storage tank, and during stable operation of the emulsifying apparatus, part of the emulsion from a primary mixing and emulsification in a first static mixer module was delivered back to a first emulsification kettle by the control of a first back flow constant-flow pump, and part of the emulsion from a secondary mixing and emulsification in a second static mixer module was delivered back to a second emulsification kettle by the control of a second back flow constant-flow pump; the flow rate of the back flow is precisely controlled by the first back flow constant-flow pump, and at the same time, the oil phase in the oil phase material storage tank and the water phase in the water phase material storage tank were continuously delivered to the first emulsification kettle by an oil phase feed constant-flow pump and a water phase feed constant-flow pump respectively, and were premixed with the emulsion being mixed and emulsified in the first emulsification kettle to obtain a premixed material, which was delivered to the first static mixer module for mixing and emulsification to obtain a premixed emulsion 1 with a water-oil ratio of 14:1;
the premixed emulsion 1 was partially delivered to the first emulsification kettle under the control of the first back flow constant-flow pump and partially delivered to the second emulsification kettle under the control of a premixed emulsion 1 feed constant-flow pump, and the premixed emulsion 1 in the premixed emulsion 1 feed constant-flow pump and the water phase continuously delivered to the second emulsification kettle by the water phase feed constant-flow pump were premixed to obtain a premixed material, which was delivered to the second static mixer module for mixing and emulsification to obtain a premixed emulsion 2; the premixed emulsion 2 was partially delivered to the second emulsification kettle under the control of the second back flow constant-flow pump and partially delivered to a third static mixer module under the control of a constant-flow pump, and was finally mixed and emulsified with the initiator water phase continuously delivered under the control of an initiator feed constant-flow pump, to obtain an HIPE emulsion 1, which was continuously delivered to the next process via an emulsified product output interface;
S2. mixing 60 parts of isooctyl methacrylate, 20 parts of ethylene glycol dimethacrylate, 1 part of a photoinitiator and 2 parts of the surfactant to obtain an oil phase 2, and then adding a water phase C containing 2% by mass of calcium chloride and a water phase D containing 5% by mass of the initiator, 0.5% by mass of sodium acrylate, 0.5% by mass of sodium methacrylate, 0.5% by mass of sodium methyl propylene sulfonate, and 0.5% by mass of sodium propylene sulfonate, with a volume ratio of the oil phase 2, the water phase C and the water phase D being 1:40:1, to obtain an HIPE emulsion 2;
the treatment process of the HIPE emulsion 2 in the continuous emulsifying apparatus was the same as that in S1;
S3. coating the HIPE emulsion 1 on the lower surface of the non-woven fabric with a thickness of 1.5 mm; coating the HIPE emulsion 2 on the upper surface of the non-woven fabric with a thickness of 0.5mm; and then using the illumination with a wavelength of 205 nm and an intensity of 1000 mW/cm² for 180 s, followed by curing in a steam tunnel furnace for 5 min, and then dehydrating, drying and slitting same to obtain the absorbent core of the liquid sanitary napkin.

### Comparative Example 1

A method for preparing an absorbent core of a liquid sanitary napkin, wherein this comparative example differs from Example 1 in that: in steps S1 and S2, the surfactant was replaced with a conventional emulsifier propyldimethylammonium bromide, and other materials and preparation processes were the same as those in Example 1.

### Comparative Example 2

A method for preparing an absorbent core of a liquid sanitary napkin, wherein this comparative example differs from Example 1 in that: 1-chlorodocosane in step S0 was replaced with n-chlorobutane, and eicosyl methacrylate in step S1 was replaced with isooctyl methacrylate, and other materials and preparation processes were the same as those in Example 1.

### Test example

A performance comparison test was performed on Example 1 and Comparative Examples 1-2.

Test methods: water absorption and blood absorption tests were performed according to GB/T14207-2008.

The test results are shown in Table 1.

**Table 1 Test results of absorption performance**

| Item | Example 1 | | | Comparative Example 1 | | | Comparative Example 2 | | |
|---|---|---|---|---|---|---|---|---|---|
| | Run 1 | Run 2 | Run 3 | Run 1 | Run 2 | Run 3 | Run 1 | Run 2 | Run 3 |
| Artificial menstrual blood penetration time (s) | 5.24 | 12.58 | 23.05 | 9.61 | 23.30 | 35.49 | 10.63 | 24.72 | 38.29 |
| | 5.77 | 12.52 | 23.08 | 9.33 | 21.71 | 38.74 | 9.97 | 22.61 | 36.24 |
| | 5.45 | 12.74 | 23.18 | 10.15 | 23.84 | 36.77 | 10.42 | 24.16 | 36.63 |
| Average | 5.49 | 12.61 | 23.10 | 9.70 | 22.95 | 37.00 | 10.34 | 23.83 | 37.05 |
| | / | / | 0.58 | / | / | 1.30 | / | / | 1.63 |
| Artificial menstrual blood rewet amount (g) | / | / | 0.69 | / | / | 1.52 | / | / | 1.54 |
| | / | / | 0.60 | / | / | 135 | / | / | 1.61 |
| Average | / | / | 0.62 | / | / | 1.39 | / | / | 1.59 |
| Water absorption amount (g) | 49.69 | 50.09 | 50.33 | 46.08 | / | / | 42.65 | / | / |
| Water absorption rate (times) | 12.30 | 12.30 | 12.60 | 11.90 | / | / | 10.80 | / | / |
| Absorption speed (s) | 26.58 | 28.95 | 28.14 | / | / | / | / | / | / |

From Table 1, it can be concluded that the compounding of the specific surfactant and the acrylic ester monomer in Example 1 of the present invention can produce a good synergistic effect, and the prepared absorbent core of the liquid sanitary napkin has excellent liquid absorption capacity, with the penetration time, rewet amount, water absorption amount and absorption speed being significantly better than those in the comparative examples. In contrast, in Comparative Examples 1-2, due to the elimination of the surfactant or the replacement of the surfactant with a short-chain structure, it is difficult for the absorbent core obtained by the reaction to form a more stereoscopic and dense microporous structure, so that the absorption capacity of the core is significantly reduced, and the ideal capillary action cannot be produced. As a result, the liquid transfer capacity in the product is poorer, and the absorption efficiency is lower.

It is apparent to those skilled in the art that the present invention is not limited to the details of the above exemplary examples, and can be implemented in other specific forms without departing from the spirit or essential characteristics of the present invention. Therefore, the examples should be considered as exemplary and non-limiting from any point of view, and the scope of the present invention is defined by the appended claims rather than the above description, and thus all changes that fall within the meaning and scope of the equivalent elements of the claims are intended to be included within the present invention.

In addition, it should be understood that although the present specification is described according to the embodiments, not each embodiment only includes one independent technical solution, and this description of the specification is only for clarity, and those skilled in the art should take the specification as a whole, and the technical solutions in the examples can also be appropriately combined to form other embodiments that can be understood by those skilled in the art.

## Claims

1. A method for preparing an absorbent core of a liquid sanitary napkin comprising the steps of:
a. mixing a monomer, a cross-linking agent, a photoinitiator, and a surfactant to form an oil phase;
b. adding a water phase to the oil phase to form a first high internal phase emulsion;
c. depositing the first high internal phase emulsion on a surface;
d. exposing the first high internal phase emulsion to a light treatment;
e. placing the first high internal phase emulsion in a steam tunnel furnace;
f. dehydrating and drying the high internal phase emulsion to obtain the absorbent core;
wherein the surfactant is obtained by reacting acrylic amino alkyl ester and/or an acrylic amino alkyl ester derivative with a haloalkane and/or a haloalkane derivative.

2. The method of claim 1, further comprising mixing a second monomer, a second cross-linking agent, a second photoinitiator, and a second surfactant to form a second oil phase; and adding a second water phase to the second oil phase to form a second high internal phase emulsion.

3. The method of claim 2, wherein the second high internal phase emulsion is deposited on the first high internal phase emulsion.

4. The method of any one of the preceding claims, wherein the alkyl group in the alkyl esters are selected from C1-C20.

5. The method of any one of the preceding claims, wherein the amino group is -N (R₁)(R₂); wherein R₁ and R₂ is independently selected from a hydrogen atom or a C1-C20 alkyl group.

6. The method of any one of the preceding claims, wherein the monomer is an acrylic ester monomer.

7. The method of any one of the preceding claims, wherein the monomer is selected from alkyl acrylate or alkyl methacrylate.

8. The method of claim 2, wherein the second monomer is selected from isooctyl acrylate or isooctyl methacrylate.

9. The method of any one of the preceding claims, wherein the light treatment comprises exposing the first high internal phase emulsion to a light having a wavelength of from 205 nm to 450 nm.

10. The method of any one of the preceding claims, wherein the water phase comprise an inorganic salt selected from one or more of alkaline earth metal chloride or sulfate and alkali metal chloride or sulfate.

11. The method of any one of the preceding claims, wherein the first high internal phase emulsion is made using a continuous emulsifying apparatus.

12. The method of any one of the preceding claims, wherein the cross-linking agent is selected from the group consisting of ethylene glycol diacrylate, ethylene glycol dimethacrylate, butylene glycol diacrylate, hexylene glycol dimethacrylate, trimethylol triacrylate, tripropylene glycol diacrylate, trimethylol trimethacrylate, tripropylene glycol dimethacrylate, and combinations thereof.

13. The method of any one of the preceding claims, wherein the oil phase comprises a mass ratio of the monomer to the cross-linking agent to the photoinitiator to the surfactant of 40-100: 5- 40: 0.05-2: 2-10.

14. The method of any one of the preceding claims, wherein the water phase comprises from 0.1 to 10 wt% of an initiator.

15. The method of claim 14, wherein the initiator is selected from one or more of ammonium persulfate, sodium persulfate, potassium persulfate, and azo initiator.

16. The method of any one of the preceding claims, wherein the photoinitiator is one or more of benzophenone, benzil, thioxanthone, benzyl ketal, α -hydroxyalkyl benzophenone, α - aminoalkylbenzophenone, and acylphosphine oxide.

17. A High Internal Phase Emulsion comprising an oil phase comprising a monomer, a cross-linking agent, a photoinitiator, and a surfactant; and a water phase; wherein the surfactant is obtained by reacting acrylic amino alkyl ester and/or an acrylic amino alkyl ester derivative with a haloalkane and/or a haloalkane derivative.

18. The High Internal Phase Emulsion of claim 17, wherein the alkyl group in the alkyl esters are selected from C1-C20.

19. The High Internal Phase Emulsion of claim 17 or 18, wherein the amino group is -N (R₁)(R₂); wherein R₁ and R₂ is independently selected from a hydrogen atom or a C1-C20 alkyl group.

20. The High Internal Phase Emulsion of any one of claims 17 to 19, wherein the monomer is an acrylic ester monomer.

21. The High Internal Phase Emulsion of any one of claims 17 to 19, wherein the monomer is selected from alkyl acrylate or alkyl methacrylate.

22. High Internal Phase Emulsion of any one of claims 17 to 21, wherein the water phase comprise an inorganic salt selected from one or more of alkaline earth metal chloride or sulfate and alkali metal chloride or sulfate.

23. The High Internal Phase Emulsion of any one of claims 17 to 22, wherein the cross-linking agent is selected from the group consisting of ethylene glycol diacrylate, ethylene glycol dimethacrylate, butylene glycol diacrylate, hexylene glycol dimethacrylate, trimethylol triacrylate, tripropylene glycol diacrylate, trimethylol trimethacrylate, tripropylene glycol dimethacrylate, and combinations thereof.

24. The High Internal Phase Emulsion of any one of claims 17 to 23, wherein the oil phase comprises a mass ratio of the monomer to the cross-linking agent to the photoinitiator to the surfactant of 40-100: 5- 40: 0.05-2: 2-10.

25. The High Internal Phase Emulsion of any one of claims 17 to 24, wherein the water phase comprises from 0.1 to 10 wt% of an initiator.
